# EUROPÄISCHE PATENTANMELDUNG

(11) **EP 0 857 731 A2**
(43) Veröffentlichungstag der Anmeldung: **12.08.1998**
(21) Anmeldenummer: 97123000.8
(22) Anmeldetag: 30.12.1997
(51) Int. Cl.: C07K 1/04, C07K 14/16, A61K 39/00, G01N 33/569

(54) **Verfahren zur Herstellung eines Peptidgemisches**

(30) Priorität: 30.12.1996 DE 19654764
(71) Anmelder: Boehringer Mannheim GmbH, 68305 Mannheim-Waldhof (DE)
(72) Erfinder: Höss, Eva Dr., 81476 München (DE); Faatz, Elke Dr., 82386 Huglfing (DE); Ofenloch-Hähnle, Beatus Dr., 82398 Polling (DE)
(74) Vertreter: Weiss, Wolfgang, Dipl.-Chem. Dr.

(57) **Zusammenfassung**

Die Erfindung betrifft ein Verfahren zur Herstellung eines Peptidgemisches, worin man Peptide an einer Festphase aus Aminosäurederivaten synthetisiert und an mindestens einer Aminosäureposition für die Kopplungsreaktion ein Gemisch von Derivaten unterschiedlicher Aminosäuren verwendet, wobei nach jedem Kopplungsschritt, in dem ein Gemisch von Derivaten unterschiedlicher Aminosäuren eingesetzt wird, ein Reaktionsschritt durchgeführt wird, um an Peptidsequenzen, an die im vorherigen Kopplungsschritt keine Aminosäure gebunden worden ist, einen selektiven Kettenabbruch zu bewirken, und das Peptidgemisch nach der Synthese von der Festphase abgespalten wird.

## Beschreibung

Die vorliegende Erfindung betrifft ein Verfahren zur Herstellung eines Peptidgemisches, das eine Vielzahl von einzelnen Peptidsequenzen umfaßt, sowie ein Verfahren zur Herstellung einer Polyhapten-Zusammensetzung unter Verwendung des Peptidgemisches. Weiterhin betrifft die Erfindung die Verwendung des Peptidgemisches bzw. der Polyhapten-Zusammensetzung in immunologischen Tests.

Der Nachweis von Immunglobulinen in Körperflüssigkeiten, insbesondere in Humanseren, wird zur Diagnostik von Infektionen mit Mikroorganismen, insbesondere Viren, wie etwa HIV, Hepatitis-Viren, etc. verwendet. Spezifische Immunglobuline in einer zu untersuchenden Probe werden üblicherweise durch Reaktion mit einem oder mehreren Antigenen, die mit den spezifischen Immunglobulinen reagieren, nachgewiesen. Verfahren zur Bestimmung von spezifischen Immunglobulinen in der Probeflüssigkeit müssen sensitiv, zuverlässig, einfach und schnell sein.

In den letzten Jahren wurden dafür zunehmend Nachweissysteme auf Basis nicht-radioaktiver Markierungsgruppen entwickelt, bei denen das Vorhandensein eines Analyten z.B. eines spezifischen Antikörpers in der untersuchten Probe mit Hilfe optischer (z.B. Lumineszenz oder Fluoreszenz), NMR-aktiver oder metallpräzipitierender Detektionssysteme bestimmt werden konnte.

EP-A-0 307 149 offenbart ein immunologisches Nachweisverfahren nach dem Brückentest-Konzept. Dabei werden zwei rekombinante Polypeptide als markiertes Antigen und als an eine Festphase bindefähiges Antigen verwendet, wobei beide rekombinanten Polypeptide den gleichen Epitopbereich umfassen, aber in unterschiedlichen Organismen exprimiert werden, um die Spezifität des Nachweises zu erhöhen. Dabei wird jeweils ein Polypeptid mit festgelegter Struktur und nicht ein Polypeptidgemisch eingesetzt.

Insbesondere auf dem Gebiet der Diagnostik von HIV-Infektionen wurden in letzter Zeit große Anstrengungen unternommen. EP-B-0 220 273 offenbart ein Verfahren zur Diagnose von HIV-Antikörpern, wobei ein oder mehrere Peptide aus unterschiedlichen kodierenden Regionen von HIV eingesetzt werden. Die Verwendung eines Peptidgemisches umfassend eine Vielzahl statistisch vorliegender Peptidsequenzen wird nicht vorgeschlagen.

US-A-5,221,610 beschreibt markierte Peptidantigene aus einem Bereich des HIV-Virus. EP-A-0 379 949 offenbart biotinylierte gp 41 und gp 32-HIV-Peptide. Die Verwendung von Peptidgemischen in immunologischen Testverfahren wird jedoch auch darin nicht beschrieben.

Die aus dem Stand der Technik bekannten immunologischen Nachweisverfahren weisen erhebliche Schwächen auf. Insbesondere findet man nur eine geringe Sensitivität, wenn zwischen dem zu bestimmenden Antikörper und dem Antigen eine relativ geringe Affinität vorliegt. Dies ist insbesondere bei einer erst seit kurzem erfolgten Serokonversion und/oder beim Auftreten neuer Subtypen eines infektiösen Mikroorganismus der Fall.

Die WO94/20521 beschreibt ein Verfahren zur Synthese von Peptidbibliotheken auf einem Cellulose-Träger. An die wachsenden Sequenzen werden an einigen Positionen Gemische von Aminosäuren gekoppelt, wodurch Sequenz-Varianten erhalten werden. Die Aminosäure-Gemische werden dabei in höchstens äquimolarer Menge bezogen auf die Zahl der Peptidkopplungsstellen eingesetzt. Nach jedem Kopplungsschritt unter Verwendung eines Aminosäure-Gemisches werden Sequenzen, an die während des Kopplungsschritts keine Aminosäure des Gemisches gebunden wurde, durch Zugabe einer weiteren Aminosäure im Überschuß abgesättigt, so daß alle Sequenzen die gleiche Länge aufweisen. Durch die Zugabe dieser weiteren Aminosäure wird jedoch keine statistische Verteilung der unterschiedlichen Peptidsequenzen erhalten, da Sequenzen mit dieser Aminosäure verstärkt vorliegen, während andere Sequenzen unterrepräsentiert sind. Die Verwendung der Peptidbibliotheken als multiple Antigene in Immunoassays wird in der WO 94/20521 nicht in Betracht gezogen.

Die Aufgabe der vorliegenden Erfindung war eine Verbesserung von immunologischen Verfahren, durch die die Nachteile des Standes der Technik zumindest teilweise beseitigt werden, und durch die bei immunologischen Nachweisverfahren, insbesondere bei erst seit kurzem erfolgten Serokonversionen und neuen Mikroorganismen-Subtypen eine ausreichende Sensitivität erzielt wird.

Die Aufgabe wird erfindungsgemäß gelöst durch ein Verfahren zur Herstellung eines Peptidgemisches, worin man Peptide an einer Festphase aus Aminosäurederivaten synthetisiert und an mindestens einer Aminosäureposition für die Kopplungsreaktion ein Gemisch von Derivaten unterschiedlicher Aminosäuren verwendet, dadurch gekennzeichnet, daß nach jedem Kopplungsschritt, in dem ein Gemisch von Derivaten unterschiedlicher Aminosäuren eingesetzt wird, ein Reaktionsschritt durchgeführt wird, um an Peptidsequenzen, an die im vorherigen Kopplungsschritt keine Aminosäure gebunden worden ist, einen selektiven Kettenabbruch zu bewirken, und das Peptidgemisch nach der Synthese von der Festphase abgespalten wird.

Überraschenderweise wurde festgestellt, daß mit dem erfindungsgemäßen Verfahren ein Peptidgemisch erhalten werden kann, das eine wesentliche Verbesserung von immunologischen Tests hinsichtlich der Subtypenerkennung, insbesondere bei Non-B-HIV-Subtypen wie etwa Subtypen A, C und D ermöglicht. Weiterhin erlaubt das erfindungsgemäße Verfahren die Synthese einer Vielzahl unterschiedlicher Peptidantigene in einem einzigen Ansatz. Darüber hinaus können auch neue Sequenzvarianten synthetisiert werden, die die Erkennung von bisher unbekannten Subtypen ermöglichen.

Bei der Kopplung in der Festphasen-Peptidsynthese wird üblicherweise der 4 bis 10-fache Überschuß an derivatisierter Aminosäure, bezogen auf die Zahl der Peptidkopplungsstellen, verwendet. Bei der erfindungsgemäßen Verwendung eines Gemisches von Aminosäurederivaten wird das Gemisch bevorzugt in höchstens äquimolarer Menge bezogen auf die Zahl der Peptidkopplungsstellen eingesetzt, d.h. die Gesamtmenge an Aminosäurederivaten ist kleiner oder gleich der Zahl der Peptidkopplungsstellen. Dadurch kann trotz der bekannten Reaktivitätsunterschiede bei der Kopplung der einzelnen Aminosäurederivate gewährleistet werden, daß alle Aminosäurederivate zu gleichen Teilen abreagieren.

Weiterhin ist das erfindungsgemäße Verfahren dadurch gekennzeichnet, daß nach jedem Kopplungsschritt, in dem ein Gemisch aus Aminosäurederivaten eingesetzt wird, ein Reaktionsschritt durchgeführt wird, um an Peptidsequenzen, an die im vorherigen Kopplungsschritt keine Aminosäure gebunden worden ist, einen selektiven Kettenabbruch zu bewirken. Dieser Abbruchschritt, der z.B. durch Endcapping oder Abstoppen durchgeführt werden kann, verhindert das Auftreten von kürzeren Sequenzen, die entstehen können, wenn aufgrund unvollständiger Kopplung in einem oder mehreren Kopplungsschritten keine Aminosäure an eine Peptidsequenz gebunden wird. Ohne Abbruchschritt könnte im nächsten Kopplungsschritt an die unvollständige Sequenz wiederum eine Aminosäure gekoppelt werden und es würden Peptidsequenzen synthetisiert werden, die lediglich um eine oder um wenige Aminosäuren kürzer sind als die gewünschten Sequenzen. Durch den Abbruchschritt wird eine weitere Kopplung an unvollständige Sequenzen verhindert, die somit leicht von den Sequenzen mit der gewünschten vollständigen Kettenlänge abgetrennt werden können. Vorzugsweise wird für die Kettenabbruchreaktion ein Säureanhydrid wie beispielsweise Essigsäureanhydrid verwendet.

Nach Beendigung der Synthese kann das Peptidgemisch entsprechend der Sequenzlänge nach an sich bekannten Verfahren, z.B. über präparative HPLC aufgereinigt werden. Bevorzugt wird das Peptidgemisch mit einer Einheitlichkeit der Kettenlänge von größer als 95%, besonders bevorzugt von größer als 98% hergestellt.

Durch das erfindungsgemäße Verfahren wird ein Gemisch der verschiedenen Peptidspezies erhalten, wobei die theoretisch sich im Versuchsansatz befindlichen Peptidsequenzen auch tatsächlich in einer Menge vorliegen, die möglichst nahe an der statistischen Menge liegt. Im Gegensatz dazu wird in WO94/20521 nach der Kopplungsreaktion mit einem Überschuß an einer weiteren einzigen Aminosäure abgesättigt, wodurch ein erhöhter Anteil an Sequenzen erzeugt wird, die diese weitere Aminosäure an der entsprechenden Position aufweisen. Ein durch das erfindungsgemäße Verfahren erhältliches Peptidgemisch, in dem die einzelnen unterschiedlichen Peptidsequenzen möglichst nahe bei der vorgegebenen statistischen Verteilung vorliegen, ist besonders zur Verwendung in einem Immunoassay geeignet, da alle theoretisch vorliegenden Sequenzen sich auch tatsächlich in ausreichender Menge in dem Gemisch befinden.

Die Festphasensynthese der Peptide kann nach an sich bekannten Verfahren durchgeführt werden wobei als Festphase besonders bevorzugt ein Harz, beispielsweise Polystyrolharz, verwendet wird.

Bei der Durchführung des erfindungsgemäßen Verfahrens wird man üblicherweise von einer bestimmten Peptidsequenz ausgehen und an mindestens einer Aminosäureposition zur Kopplung anstelle eines einzigen Aminosäurederivats ein Gemisch aus mindestens zwei Aminosäurederivaten verwenden. Auf diese Weise können beliebige Variationen der Sequenz erhalten werden. Bevorzugt werden Sequenzen synthetisiert, die einen immunologisch reaktiven variablen Epitopbereich umfassen. Dieser Epitopbereich kann beispielsweise von einem Pathogen, einem Autoantigen, einem Tumorantigen oder einem Allergen stammen. Besonders bevorzugt wird eine Sequenz mit einem viralen Epitopbereich synthetisiert.

An die Positionen, an die ein Gemisch von Derivaten unterschiedlicher Aminosäuren gekoppelt wird, kann ein Gemisch von Aminosäuren ausgewählt aus bekannten Varianten des Epitopbereichs, ein Gemisch von Aminosäuren ausgewählt aus bekannten Varianten und willkürlich ausgewählten Aminosäuren oder/und ein Gemisch von willkürlich ausgewählten Aminosäuren gekoppelt werden.

Bei der Herstellung des Peptidgemisches werden bevorzugt an denjenigen Positionen, die anhand von bekannten Subtypen-Varianten als variabel bekannt sind, bei der Kopplung Gemische aus Derivaten von unterschiedlichen Aminosäuren verwendet.

Es ist jedoch auch erfindungsgemäß möglich, neue Peptidsequenzen herzustellen, indem die Positionen- oder/und das Aminosäuregemisch zufällig ausgewählt werden.

Es ist bevorzugt, ein Peptidgemisch herzustellen, worin die einzelnen Peptidsequenzen neben dem reaktiven Epitopbereich bzw. Variationen davon einen Spacerbereich umfassen. Der Spacerbereich ist vorzugsweise eine immunologisch inaktive Peptidsequenz mit einer Länge von 1 bis 20, besonders bevorzugt von 1 bis 10 Aminosäuren. Die Aminosäuren des Spacerbereichs werden vorzugsweise ausgewählt aus der Gruppe bestehend aus Glycin, β-Alanin, γ-Aminobuttersäure, ε-Aminocapronsäure und Lysin. Der Spacerbereich ist vorzugsweise eine kontinuierliche Abfolge von Aminosäuren am Aminoterminus oder/und Carboxyterminus des Epitopbereichs.

Es können Peptidsequenzen beliebiger Länge gemäß dem erfindungsgemäßen Verfahren hergestellt werden. Bevorzugt weisen die synthetisch hergestellten Peptidsequenzen eine Länge von 6 bis 50 Aminosäuren auf.

Durch das erfindungsgemäße Verfahren werden vorzugsweise Peptidgemische synthetisiert, die von einer Sequenz ausgehen, die einen Epitopbereich aus pathogenen Organismen, z.B. Bakterien, Viren und Protozoen oder aus Autoimmun-Antigenen umfaßt. Vorzugsweise stammt der reaktive Epitopbereich aus viralen Antigenen, z.B. den Aminosäuresequenzen von HIV-I, HIV-Subtyp 0, HIV-II oder Hepatitis C-Virus (HCV).

Vorzugsweise wird als Sequenz ein HIV-I- bzw. HIV-II-Epitop aus den Regionen gp32, gp41 und gp120 ausgewählt. HCV-Epitope werden vorzugsweise aus der Core-Env-Region oder den Nicht-Strukturprotein-Regionen NS3, NS4 oder NS5 ausgewählt.

Besonders bevorzugt wird der Epitopbereich der Sequenz von HIV-I, HIV-Subtyp 0- oder HIV-II-Aminosäuresequenzen ausgewählt aus der Gruppe der Aminosäuresequenzen: 
NNTRKSISIG PGRAFYT (I)
NTTRSISIGP GRAFYT (II)
QIDIQEMRIG PMAWYS (III)
QARILAVERY LKDQQLLGIW GASG (IV)
LGIWGCSGKL ICTTAVPWNA SWS (V)
KDQQLLGIWG SSGKL (VI)
ALETLLQNQQ LLSLW (VII)
LSLWGCKGKL VCYTS (VIII)
WGIRQLRARL LALETLLQN (IX) und
QAQLNSWGCA FRQVCHTTVP WPNDSLT (X)
 oder Teilsequenzen davon, die eine Länge von mindestens 6 Aminosäuren und vorzugsweise mindestens 8 Aminosäuren aufweisen. Die Aminosäuresequenzen I bis III stammen aus der gp120-Region von HIV-I, die Aminosäuresequenzen IV bis IX stammen aus der gp41-Region von HIV-I und die Aminosäuresequenz X stammt aus der gp32-Region von HIV-II. Die Sequenzen IV, VIII und X erhalten jeweils zwei Cysteine, die vorzugsweise in Form einer Disulfidbrücke vorliegen. Vorzugsweise enthalten die Sequenzen einen N- oder/und C-terminalen Spacer, wie oben definiert, der gegebenenfalls mindestens eine Markierung, S-, Aktivierungs- oder Festphasenbindegruppe trägt, vorzugsweise Biotin, ein Biotinanalogon, ein Metallchelat, wie etwa BPRu, oder einen Cysteinrest. Gegebenenfalls können auch innerhalb des Epitopbereichs liegende Lysinreste in markierter Form vorliegen.

Besonders bevorzugt handelt es sich bei dem erfindungsgemäßen Peptidgemisch um ein Gemisch aus Peptiden auf Basis der obigen Aminosäuresequenzen (I) oder/und (II) oder auf Basis der Aminosäuresequenz (IV) und Variationen davon.

Mit dem erfindungsgemäßen Verfahren können sowohl lineare und cyclische als auch verzweigte Peptidsequenzen hergestellt werden. Verzweigte Peptide werden bevorzugt dadurch hergestellt, daß in die Peptidsequenzen Verzweigungsstellen eingebaut werden. Solche Verzweigungsstellen können beispielsweise innerhalb immunologisch nicht reaktiver Spacerabschnitte eingebaut werden und aus tri- oder höherfunktionellen Linkermolekülen bestehen. Geeignete trifunktionelle Linkermoleküle sind beispielsweise trifunktionelle Aminosäuren, wie z.B. Lysin oder Ornithin.

Anhand der Zahl der Verzweigungsstellen oder/und anhand der Zahl der funktionellen Gruppen der Linkermoleküle kann der Grad der Verzweigung festgelegt werden. Solche verzweigten Peptidgemische werden auch als multimere Antigenzusammen-setzungen bezeichnet und sind z.B. in der deutschen Patentanmeldung DE 44 30 972 näher beschrieben.

Die Antigene der erfindungsgemäßen multimeren Antigen-zusammensetzungen weisen bevorzugt die allgemeine Formel P¹{P²[P³(P⁴)ₜ]ₛ}ᵣ auf, wobei P¹, P², P³ und P⁴ Peptidsequenzen des erfindungsgemäß hergestellten Peptidgemisches mit einer Länge der von 6 bis 50 Aminosäuren bedeuten, wobei in den Antigenen mindestens 2 Peptidsequenzen mit gleichen oder verschiedenen immunologisch reaktiven Epitopbereichen vorliegen, r 1 oder 2 ist, s eine ganze Zahl von 0 bis 4 ist, und t eine ganze Zahl von 0 bis 8 ist. Bevorzugt erhalten die Antigene mindestens eine Verzweigunsstelle. Die Peptidgemische P¹, P², P³ und P⁴ umfassen dabei jeweils eine Vielzahl unterschiedlicher, statistisch vorliegender Sequenzen, wodurch erfindungsgemäße multimere Antigenzusammensetzungen eine Vielzahl unter-schiedlicher multimerer Antigene umfassen und somit eine Vielzahl von Epitopbereichen aufweisen.

Weiterhin können multimere Antigen-Zusammensetzungen auch hergestellt werden, indem man jeweils mindestens zwei Peptide eines erfindungsgemäßen Peptidgemisches kovalent miteinander verknüpft. Dabei können mehrere Epitopbereiche kovalent über Spacerbereiche miteinander gekoppelt werden. Vorzugsweise erfolgt die Verknüpfung der Epitope mindestens teilweise über trifunktionelle Linkermoleküle, so daß die Antigen-Zusammensetzung mindestens eine Verzweigungsstelle und vorzugsweise ein bis sieben Verzweigungsstellen umfaßt. Solche multimeren Antigene bilden eine baumarige Struktur mit Verzweigungsstellen und enthalten vorzugsweise immunologisch reaktive Epitopbereiche.

Die Peptide der erfindungsgemäßen Peptidgemische enthalten vorzugsweise jeweils mindestens eine Markierungs-, Aktivierungs- oder Festphasenbindegruppe. Diese Gruppen können beispielsweise selektiv an die Enden oder/und an reaktive Seitenketten der Peptidsequenzen oder/und an Spacerabschnitte gekoppelt werden.

Die Peptide des Peptidgemisches können mit allen bekannten Markierungsgruppen, Aktivierungsgruppen oder Festphasenbindegruppen gekoppelt werden. Bevorzugt wird mindestens eine Markierungsgruppe, Aktivierungsgruppe oder Festphasenbindegruppe an einen Spacerbereich der Peptide gekoppelt.

Als Markierungsgruppen können radioaktive oder nichtradioaktive Markierungsgruppen verwendet werden, wobei nichtradioaktive Markierungsgruppen bevorzugt sind. Die Markierungsgruppen können direkt oder/und indirekt nachweisbar sein. Bei einer indirekt nachweisbaren Markierung werden die Peptide mit einer selbst nich direkt nachweisbaren Gruppe gekoppelt, z.B. einer Biotin- oder Haptengruppe, die durch Reaktion mit einem geeigneten Bindepartner (Streptavidin, Avidin bzw. Anti-Hapten-Antikörper), der eine signalerzeugende Gruppe trägt, nachweisbar ist.

Die Peptide können auch mit direkt nachweisbaren Markierungsgruppen gekoppelt werden. Beispiele für solche direkt signalerzeugenden Gruppen sind Chromogene (fluoreszierende oder lumineszierende Gruppen, Farbstoffe), Enzyme, NMR-aktive Gruppen oder Metallpartikel, die auf bekannte Weise an das Peptidgemisch gekoppelt werden. Vorzugsweise ist die direkt nachweisbare Markierungsgruppe ein durch Elektrochemolumineszenz nachweisbares Metallchelat, ausgewählt aus der Gruppe bestehend aus Ruthenium-, Rhenium-, Irridium- und Osmiumchelaten. Geeignete Liganden sind beispielsweise aromatische heterozyklische Polydentatliganden und bevorzugt Bipyridyl-, Bipyrazyl-, Terpyridyl- und Phenanthrolylliganden. Besonders bevorzugt ist Bispyridyl-Ruthenium (BPRu). Ein Verfahren zur Herstellung von Metallchelat-markierten Peptiden ist beispielsweise in der deutschen Patentanmeldung DE 44 30 998 A1 beschrieben.

Als Aktivierungsgruppen werden im allgemeinen solche Gruppen verwendet, die eine chemische kovalente Kopplung des Peptids an einen Bindepartner, z. B. ein Tragermolekül ermöglichen. Beispiele für Aktivierungsgruppen sind SH-Gruppen, die durch Cysteinreste in das Peptid eingeführt werden können. Diese SH-Gruppen können mit SH-reaktiven Gruppen auf dem Träger, z.B. Maleinimidgruppen, gekoppelt werden. Andererseits können auch Maleinimidgruppen in das Peptid eingeführt werden, welche dann mit SH-Gruppen des Trägers reagieren können. Weiterhin können in das Peptid auch Aktivester, z.B. N-Hydroxysuccinimid eingeführt werden, die mit NH₂-Gruppen eines Trägers gekoppelt werden können.

Als Festphasenbindegruppen werden in das Peptid vorzugsweise solche Gruppen eingeführt, die ein hochaffine nicht-kovalente Wechselwirkung mit einer Festphase eingehen. Beispiele für geeignete Festphasenbindegruppen sind Haptene, die mit einer Anti-Hapten-Antikörper-beschichteten Festphase gekoppelt werden können. Bevorzugt als Festphasenbindegruppen sind Biotin oder Biotinanaloga, wie z.B. Iminobiotin, Aminobiotin, Desthiobiotin, die eine Kopplung an eine Streptavidin-beschichtete Festphase erlauben.

Ein weiterer Gegenstand der vorliegenden Erfindung ist ein durch das obige Verfahren erhältliche Peptidgemisch, in dem die Peptide in freier Form vorliegen. Dieses Peptidgemisch enthält mehrere Peptide, die vorzugsweise eine Länge von 6 bis 50 Aminosäuren aufweisen, und die jeweils an selektiv ausgewählten Positionen mindestens eine Gruppe ausgewählt aus Markierungs-, Aktivierungs- und Festphasenbindegruppen enthalten.

Noch ein weiterer Gegenstand der vorliegenden Erfindung ist ein Peptidgemisch, das mehrere Peptide, die vorzugsweise eine Länge von 6 bis 50 Aminosäuren aufweisen, enthält, wobei die Peptide Varianten eines immunologisch reaktiven Epitopbereichs darstellen und jeweils selektiv ausgewählten Positionen mindestens eine Gruppe ausgewählt aus Markierungs- , Aktivierungs- und Festphasenbindegruppen enthalten.

Dabei bedeutet das Vorhandensein der Markierungs-, Aktivierungs- und Festphasenbindegruppen an selektiv ausgewählten Positionen, daß die Einführung dieser Gruppen gezielt durch die chemische Synthese erfolgt. Die einzelnen Peptide des Gemisches weisen vorzugsweise eine Sequenzhomologie von jeweils mindestens 30% zu einer immunologisch reaktiven Basissequenz auf, d. h. zu einer natürlich vorkommenden Peptidsequenz eines Antigens, bzw. einer aus mehreren Subtypen ermittelten Antigenkonsensussequenz auf. Das Peptidgemisch besteht bevorzugt aus 2 bis 2000 einzelnen Spezies und besonders bevorzugt aus mindestens 3, 4 oder 10 und aus höchstens 1000 einzelnen Spezies. Das Peptidgemisch kann aber auch aus bis zu 10¹⁰ einzelnen Spezies bestehen.

Das erfindungsgemäße Peptidgemisch umfaßt vorzugsweise einen immunologisch reaktiven Epitopbereich oder Variationen davon, der mit Antikörpern, z.B. aus Humanseren reagieren kann und einen immunologisch nicht-reaktiven Spacerbereich, wobei der Spacerbereich bevorzugt die mindestens eine Markierungsgruppe, Aktivierungsgruppe oder Festphasenbindegruppe trägt. Vorzugsweise ist der Spacerbereich am Aminoterminus der Peptide angeordnet und hat eine Länge von 1 bis 20, besonders bevorzugt von 1 bis 10 Aminosäuren. Der Epitopbereich wird vorzugsweise aus viralen Epitopbereichen, insbesondere aus HIV-I, HIV-Subtyp 0 oder HIV-II einschließlich Varianten davon ausgewählt und ist besonders bevorzugt eine der Aminosäuresequenzen (I) bis (XI) oder eine Teilsequenz davon. Am meisten bevorzugt stammen die Epitopbereiche aus gp41 oder gp120 von HIVI oder/und HIV Subtyp O.

An die Peptide des Peptidgemisches ist weiterhin bevorzugt mindestens eine Markierungsgruppe, mindestens eine Aktivierungsgruppe oder mindestens eine Festphasebindegruppe gekoppelt. Beispielsweise kann am Aminoterminus oder/und an Aminoseitengruppen mindestens 1 Hapten gekoppelt sein. Weiterhin können die Peptide des Peptidgemisches an ein Metallchelat wie oben beschrieben oder an Biotin oder Biotinanaloga gekoppelt sein.

Ein weiterer Gegenstand der Erfindung ist ein Verfahren zur Herstellung einer Polyhapten-Zusammensetzung, dadurch gekennzeichnet, daß man ein erfindungsgemäßes Peptidgemisch z. B. über eine Aktivierungsgruppe mit einem Träger koppelt. Die Zahl der Peptidsequenzen pro Träger beträgt größer 1 bis 100 und bevorzugt größer 1 bis 40. Es werden dabei Polyhaptene gebildet, die eine große Variation von Epitopbereichen aufweisen. Als Träger sind mit dem zu bestimmenden Antikörper nicht reagierende Substanzen, insbesondere Makromoleküle geeignet, an die die Epitopbereiche kovalent gekoppelt werden. Beispiele für geeignete Träger sind Peptide, Polypeptide oder synthetische Träger z.B. Dextrane. Beispiele für geeignete Polypeptide sind Albumine, z.B. Rinderserum-Albumin, unspezifische Immunglobuline, Immunglobulinfragmente, β-Galactosidase und Polylysin. Bei der Verwendung eines inerten Trägers ist darauf zu achten, daß er keine Kreuzreaktivität mit Antikörpern in der Probenflüssigkeit zeigt.

Die Epitopbereiche werden vorzugsweise über einen bifunktionellen Linker an reaktive Gruppen des Trägers, z.B. NH₂- oder SH-Gruppen gekoppelt. Vorzugsweise erfolgt die Kopplung über NH₂-Gruppen des Trägers. Ein geeignetes Verfahren zur Herstellung von Polyhaptenen ist beispielsweise in der deutschen Patentanmeldung DE 44 30 972 A1 beschrieben.

Die Anzahl der Markierungs- oder Festphasenbindegruppen der Polyhaptene ist variabel, d.h. es können eine oder mehrere Gruppen vorhanden sein. In manchen Ausführungsformen des erfindungsgemäßen Verfahrens ist es bevorzugt, wenn mindestens drei und besonders bevorzugt drei bis 20 Markierungs- oder Festphasebindegruppen vorhanden sind. Die Festphasenbindegruppen oder Markierungsgruppen können dabei sowohl an die Peptide als auch direkt an den Träger gekoppelt sein.

Die erfindungsgemäß hergestellten Polyhapten-Zusammensetzungen ermöglichen die Erkennung von Antikörpern verschiedenster Subtypen in Serokonversionsproben, ohne daß man auf die Grenzen der Wandbeladung des Probegefäßes stößt.

Ein weiterer Gegenstand der Erfindung ist deshalb eine Polyhapten-Zusammensetzung, die durch das zuvor beschriebene Verfahren erhältlich ist.

Noch ein weiterer Gegenstand der Erfindung ist eine Polyhapten-Zusammensetzung, umfassend einen Träger und daran kovalent gekoppelt ein Gemisch von Peptiden, wobei die Peptide Varianten eines immunologisch reaktiven Epitopbereichs darstellen und über eine selektiv ausgewählte Position mit dem Träger gekoppelt sind.

Vorzugsweise enthält die Polyhaptenzusammensetzung 2 bis 2000 und besonders bevorzugt 3 bis 1000 einzelne Peptidspezies gekoppelt an Trägermoleküle. Die Länge der Peptidsequenzen beträgt vorzugsweise 6 bis 50 Aminosäuren.

Die Kopplung der Peptide kann über den N-Terminus, den C-Terminus oder über reaktive Gruppen in der Seitenkette an den Träger erfolgen. Eine Möglichkeit der Kopplung besteht darin, die Trägermoleküle durch Reaktion mit bekannten Linkersubstanzen (z.B. Maleinimidohexansäure, Maleinimido-propionsäure, Maleinimidobenzoesäure) an einer NH₂-Gruppe zu aktivieren und ein SH-aktiviertes Peptidgemisch kovalent an den Träger zu koppeln. Die Markierungs- oder Festphasenbindegruppen werden üblicherweise in Form von Aktivestern an das Trägermolekül oder/und an die Epitopbereich gekoppelt. Es sind aber auch andere Kopplungsmöglichkeiten, z.B. über bifunktionelle Photolinker denkbar (siehe z.B. deutsche Patentanmeldung DE 44 30 972).

Die Polyhaptene weisen bevorzugt die allgemeine Formel (P-)ₙT(-L)ₘ oder T(-P-Lₘ)ₙ auf, wobei T einen Träger bedeutet, P Peptidsequenzen des erfindungsgemäßen Peptidgemisches darstellt, die kovalent an den Träger gekoppelt sind und L Markierungsgruppen oder Festphasebindegruppen darstellt, die kovalent an den Träger bzw. die Peptidsequenzen gekoppelt sind, n eine Zahl von größer 1 bis 100, bevorzugt größer 1 bis 40 ist und m eine Zahl von 1 bis 10 ist, bevorzugt ≥ 2 ist.

Die erfindungsgemäßen Polyhapten-Zusammensetzungen umfassen eine Vielzahl von unterschiedlichen Polyhaptenen, die die Epitopbereiche der statistisch verteilten Sequenzen des Peptidgemisches enthalten.

Die oben beschriebenen Peptidgemische, Polyhapten-Zusammensetzungen und multimeren Antigen-Zusammensetzungen können erfindungsgemäß als immunologisches Nachweisreagenz, insbesondere zur Bestimmung von Antikörpern in einer Probenflüssigkeit eingesetzt werden. Bevorzugt werden die Substanzen bei einem immunologischen Verfahren im Brückentest, und besonders bevorzugt als multiples Antigen zur Verbesserung der Subtypenerkennung verwendet.

Weiterhin umfaßt die Erfindung ein Verfahren zur immunologischen Bestimmung eines Analyten, insbesondere eines Antikörpers in einer Probenflüssigkeit, das dadurch gekennzeichnet ist, daß man die Probenflüssigkeit mit einem Peptidgemisch oder/und einer Polyhapten-Zusammensetzung kontaktiert, wobei das Peptidgemisch oder/und das Polyhapten Peptide umfaßt, die spezifisch an den zu bestimmenden Analyten binden, und den Analyten über die Bildung von Immunkomplexen qualitativ oder/und quantitativ nachweist.

Die Menge des Peptidgemisches bzw. der Polyhaptenzusammensetzung bei der Bestimmung des Analyten kann je nach Testformat variiert werden. Die für ein Testformat optimale Einsatzmenge ist mit einem repräsentativen Serenkollektiv ohne weiteres zu ermitteln. Die verwendete Menge kann dabei der für ein Einzelantigen verwendeten Menge entsprechen. In vielen Testformaten sind jedoch erhöhte Einsatzmengen günstiger, z. B. eine Einsatzmenge, die mindestens einen Faktor 5, z. B. einen Faktor 10 über der optimalen Einsatzmenge eines Einzelantigens liegt. Die absolute Menge des Antigens hängt vom Testformat ab.

Noch ein weiterer Gegenstand der Erfindung betrifft ein Reagenz zur immunologischen Bestimmung eines Analyten, insbesondere eines Antikörpers, das dadurch gekennzeichnet ist, daß es mindestens ein gegen den zu bestimmenden Analyten gerichtetes Peptidgemisch oder/und Polyhapten umfaßt.

Die Erfindung wird durch die nachfolgenden Beispiele näher beschrieben.

### Beispiele:

### Beispiel 1:

### Biotinylierte, Bipyridyl-Ruthenium-gelabelte oder Thiol-aktivierte Peptide

Unter dem Begriff Label werden in den folgenden Beispielen Bipyridyl-Ruthenium-Komplexe sowie Biotin zusammengefaßt. Weiterhin sind darunter allgemein Fluorophore, Chemiluminophore, Chromophore, Enzyme, Radionucleotide sowie Partikel (z.B. Magnetpartikel, Gold) zu verstehen. Als Spacer werden bei den Peptiden stabilisierende und löslichkeitsvermittelnde Spacer, die entweder Ladungen enthalten und/oder Wasserstoffbrücken ausbilden können, eingesetzt. Zumeist handelt es sich um Kombination aus natürlichen und/oder artifiziellen Aminosäure-Derivaten (z.B. Kombination aus ε-Aminocapronsäure, β-Alanin, γ-Aminobuttersäure und/oder Lysin). Als Epitop wird eine reaktive Peptidsequenz oder ein Protein, das mit Humanseren-Antikörpern reagiert bezeichnet Als Träger wird ein Protein (z.B. RSA, β-Gal, div. IgGs) oder ein synthetischer Träger (z.B. Aminodextran) verwendet.

Die entsprechenden Teilsequenzen der Aminosäuresequenz des HIV-Virusproteins werden mittels Fluorenylmethyloxycarbonyl(Fmoc)-Festphasenpeptidsynthese an einem Batch-Peptidsynthesizer, z.B. von Applied Biosystems A431 oder A433, hergestellt. Dazu werden folgende Fmoc-Aminosäurederivate verwendet:

| | |
|---|---|
| A | Fmoc-Ala-OH |
| C | Fmoc-Cys(Trt)-OH |
| D | Fmoc-Asp(tBu)-OH |
| E | Fmoc-Glu(tBu)-OH |
| F | Fmoc-Phe-OH |
| G | Fmoc-Gly-OH |
| H | Fmoc-His(Trt)-OH |
| I | Fmoc-Ile-OH |
| K | Fmoc-Lys(Boc)-OH |
| L | Fmoc-Leu-OH |
| M | Fmoc-Met-OH |
| N | Fmoc-Asn(Trt)-OH |
| Nle | Fmoc-Norleucin |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBu)-OH |
| U | Fmoc-βAlanin |
| V | Fmoc-Val-OH |
| W | Fmoc-Trp-OH |
| X | Boc-Lys(Fmoc)-OH |
| Y | Fmoc-Tyr(tBu)-OH |
| Z | Fmoc-ε Aminocapronsäure |

Bei den jeweiligen Synthesezyklen werden verschiedene Mischungen an Aminosäuren eingesetzt. Soll nur eine definierte Aminosäure gekuppelt werden, verwendet man 4 Äquivalente an Fmoc-geschütztem Aminosäurederivat, soll eine Mischung von Aminosäuren gekuppelt werden wird nur die äquimolare Menge an Aminocarbonsäure verwendet. Dies hat den Vorteil, daß alle Aminosäure mit gleicher Wahrscheinlichkeit gekuppelt werden und so eine repräsentative Mischung erhalten wird.

Bei cyclischen Peptiden wird die Aminosäure Methionin gegen Norleucin ersetzt, um Oxidationsprodukte bei der Cyclisierung des Loops zu vermeiden.

Die Aminosäuren oder Aminosäurederivate werden in N-Methylpyrrolidon gelöst. Das Peptid wird an 400-500 mg 4-(2',4'-Dimethoxyphenyl-Fmoc-Aminomethyl)-Phenoxy-Harz (Tetrahedron Letters 28 (1987), 2107) mit einer Beladung von 0,4 - 0,7 mmol/g aufgebaut (JACS 95 (1973), 1328). Die Kupplungsreaktionen werden bezüglich Fmoc-Aminosäurederivat mit 1 oder 4 Äquivalenten Dicyclohexylcarbodiimid und 1 oder 4 Äquivaltenten N-Hydroxybenzotriazol in Dimethylformamid als Reaktionsmedium während 20 min durchgeführt. Nach jedem Kupplungsschritt werden nicht abreagierte Aminogruppen mit Essigsäureanhydrid gecapped. Anschließend wird die Fmoc-Gruppe mit 20 %igem Piperidin in Dimethylformamid in 20 min abgespalten. Enthalten die Peptide eine intramolekulare Disulfidbrücke, so wird die Fmoc-geschützte Peptidsequenz vor der Spacersynthese mit Jod in Hexofluorisopropanol (Kober et al., The Peptide Academic Press, New York, 1981, Seiten 145-47) an der Festphase oxidiert. Im Anschluß wird erneut die N-terminale Fmoc-Schutzgruppe abgespalten und die Spacer-Aminosäuren sowie gegebenenfalls der Label mit dem Standardverfahren gekuppelt.

Die Freisetzung des Peptides vom Syntheseharz und die Abspaltung der säurelabilen Schutzgruppen erfolgt mit 20 ml Trifluoressigsäure, 0,5 ml Ethandithiol, 1 ml Thioanisol, 1,5 g Phenol und 1 ml Wasser in 40 min bei Raumtemperatur. Die Reaktionslösung wird anschließend mit 300 ml gekühltem Diisopropylether versetzt und zur vollständigen Fällung des Peptides 40 min bei 0°C gehalten. Der intensiv gelb oder orange gefärbte Niederschlag wird abfiltriert, mit Diisopropylether nachgewaschen, mit wenig 50 %iger Essigsäure gelöst und lyophilisiert. Vom erhaltene Rohmaterial werden mittels präparativer HPLC an Delta-PAK RP C18 (Säule 50 x 300 mm, 100 Å, 15 µ) über einen entsprechenden Gradienten (Eluent A: Wasser, 0,1 % Trifluoressigsäure, Eluent B: Acetonitril, 0,1 % Trifluoressigsäure) in ca. 120 min kürzere, acetylierte Abbruchsequenzen abgetrennt. Die Identität des eluierten Materials wird mittels HPLC-Massenspektrometrie geprüft.

In analoger Weise lassen sich auch multimere Antigene synthetisieren.

### Beispiel 2:

Folgende Peptidgemische wurden unter Verwendung der angegebenen Aminosäurederivate entsprechend Beispiel 1 hergestellt und immunologisch bewertet.

Die immunologische Bewertung der Peptidgemische erfolgte analog dem Boehringer Mannheim Enzymun-Test Anti-HIV1+2 (Boehringer Mannheim GmbH, Kat.-Nr. 1 165062). Das Testprinzip war ein Zweischritt-ELISA mit einer Streptavidin Festphase. Jedoch wurde statt des biotinylierte Antigens eine erfindungsgemäße Peptidmischung in der angegebenen Konzentration eingesetzt. Die Messungen wurden an den Enzymun-Analyseautomaten ES22 oder ES600 von Boehringer Mannheim durchgeführt.

### a) Gemisch 1: Random-Peptid HIV,gp120 V3

| Bi | Spacer | N | N | T | R | K | S | I | S | I | G | P | G | R | A | F | Y | T |
|---|---|---|---|---|---|---|---|---|---|---|---|---|---|---|---|---|---|---|
| | | | | | | R | | | | | | | | Q | V | | | A |
| | | | | | | | | | | | | | | | T | | | |
| | | | | | | | | | | | | | | | | | | |
| | | | | | | | | | | | | | | | | | | |
| | | | | | | | | | | | | | | | | | | |
| | | | | | | | | | | | | | | | | | | |
| | | | | | | | | | | | | | | | | | | |

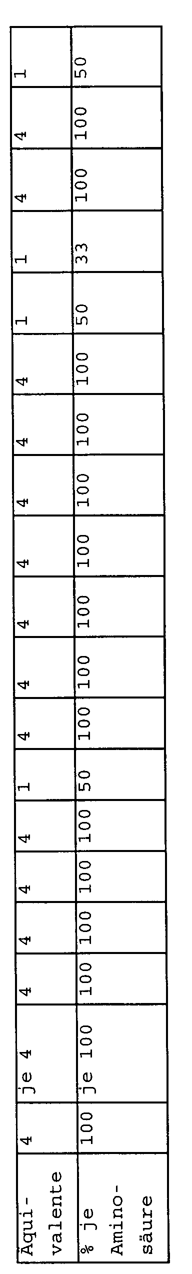

In der ersten Zeile ist die Aminosäuresequenz des Standardantigens 1 aus HIV gp120 V3 angegeben. Die Spacersequenz ist XUZU. Das erhaltene Peptidgemisch umfaßt 24 unterschiedliche Peptidsequenzen.

### b) Gemisch 2: Random-Peptid HIV,gp120 V3

| Bi | Spacer | N | N | T | R | K | S | I | S | I | G | P | G | R | A | F | Y | T |
|---|---|---|---|---|---|---|---|---|---|---|---|---|---|---|---|---|---|---|
| | | | | | | R | R | T | H | | | | | Q | V | | | A |
| | | | | | | Q | | V | P | | | | | | T | | | |
| | | | | | | | | | | | | | | | | | | |
| | | | | | | | | | | | | | | | | | | |
| | | | | | | | | | | | | | | | | | | |
| | | | | | | | | | | | | | | | | | | |
| | | | | | | | | | | | | | | | | | | |

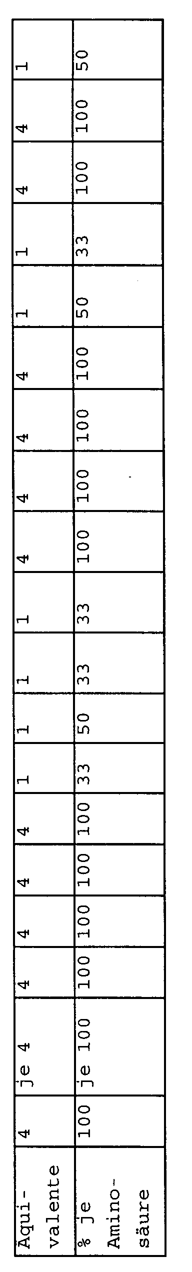

In der ersten Zeile ist die Aminosäuresequenz des Standardantigens 1 aus HIV gp120 V3 angegeben. Die Spacersequenz ist XUZU. Das erhaltene Peptidgemisch umfaßt 648 unterschiedliche Peptidsequenzen.

### c) Gemisch 3: Random-Peptid HIV,gp41

| Bi | Spacer | A | V | E | R | Y | L | K | D | Q | Q | L | L | G | I | W |
|---|---|---|---|---|---|---|---|---|---|---|---|---|---|---|---|---|
| | | | L | | S | | I | R | | | K | F | | N | L | |
| | | | | | | | | Q | | | | | | | | |
| | | | | | | | | | | | | | | | | |

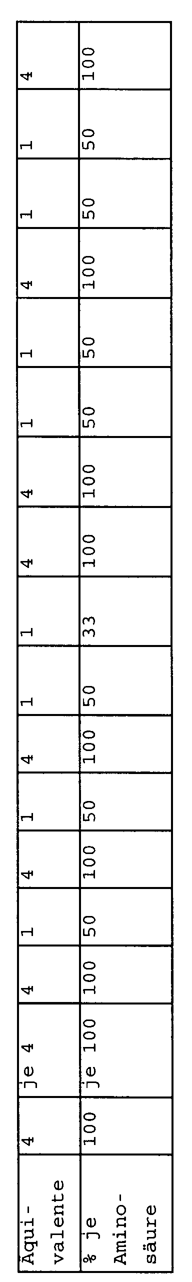

In der ersten Zeile ist die Aminosäuresequenz des Standardantigens 2 aus HIV gp41 angegeben. Die Spacersequnez ist XUZU.Das erhaltene Peptidgemisch umfaßt 384 unterschiedliche Peptidsequenzen.

Die Ergebnisse mit den erfindungsgemäßen Peptidgemischen waren wie folgt.

Bei einigen Seren verschiedener Subtypen ist bereits bei gleicher Einsatzmenge die Erkennung durch das Peptidgemisch höher als mit der B-Sequenz des Standardantigens 1 (Konsensussequenz für Subtyp B).

| **Serum** | **Subtyp** | **Standardantigen 1** | **Gemisch 1** | **Gemisch 2** |
|---|---|---|---|---|
| | | Einsatz 200 ng/ml | Einsatz 200 ng/ml | Einsatz 200 ng/ml |
| | | Signal in mE | Signal in mE | Signal in mE |
| Negativkontrolle | | 158 | 126 | 137 |
| Positivserum 1 | C | 2199 | 2417 | 2495 |
| Positivserum 2 | A | 1537 | 1572 | 1739 |

Bei anderen Seren wird bei einer Erhöhung der Einsatzmenge eine Verbesserung des Testergebnisses durch Verwendung des Gemisches gefunden.

| **Serum** | **Subtyp** | **Standardantigen 1** | **Gemisch 1** | **Gemisch 2** |
|---|---|---|---|---|
| | | Einsatz 20 ng/ml | Einsatz 200 ng/ml | Einsatz 200 ng/ml |
| Negativkontrolle | | 158 | 126 | 137 |
| Positivserum 3 | B | 2037 | 2994 | 2640 |
| Positivserum 4 | B | 4932 | 4602 | 4968 |
| Positivserum 5 | B | 729 | 850 | 1054 |
| Positivserum 6 | B | 519 | 610 | 864 |
| Positivserum 7 | B | 3523 | 4289 | 3654 |
| Positivserum 8 | C | 703 | 2417 | 2495 |
| Positivserum 9 | C | 1290 | 1288 | 1852 |
| Positivserum 10 | A | 224 | 1572 | 1739 |
| Positivserum 11 | C | 336 | 1240 | 626 |
| Positivserum 12 | C | 511 | 1543 | 903 |
| Positivserum 13 | CA | 1939 | 2448 | 3092 |
| Positivserum 14 | D | 345 | 3799 | 3348 |
| Positivserum 15 | AC | 2236 | 4091 | 4275 |

Auch bei Standardantigen 2 (Konsensussequenz für Subtyp B) ist die Erkennung durch das Peptidgemisch bereits bei gleichen Einsatzmengen höher.

| **Serum** | **Subtyp** | **Standardantigen 2** | **Gemisch 3** |
|---|---|---|---|
| | | Einsatz 200 ng/ml | Einsatz 200 ng/ml |
| | | Signal in mE | Signal in mE |
| Negativkontrolle | | 105 | 110 |
| Positivserum 12 | C | 1877 | 2961 |
| Positivserum 15 | D | 123 | 1344 |
| Positivserum 16 | C | 291 | 1492 |

Bei anderen Seren muß die Einsatzmenge erhöht werden, um eine Verbesserung durch das Peptidgemisch zu erreichen.

| **Serum** | **Subtyp** | **Standardantigen 2** | **Gemisch 3** |
|---|---|---|---|
| | | Einsatz 20 ng/ml | Einsatz 200 ng/ml |
| | | Signal in mE | Signal in mE |
| Negativkontrolle | | 100 | 110 |
| Positivserum 3 | B | 141 | 314 |
| Positivserum 4 | B | 254 | 1010 |
| Positivserum 5 | B | 3313 | 3613 |
| Positivserum 8 | C | 1173 | 1310 |
| Positivserum 11 | C | 340 | 2961 |
| Positivserum 12 | D | 187 | 283 |
| Positivserum 14 | D | 66 | 1344 |
| Positivserum 15 | AC | 116 | 1492 |

### Beispiel 3:

### Polyhaptene

Für die Synthese der Polyhaptene (PH) werden die entsprechenden Peptide mit einer reaktiven Mercaptofunktion, z.B. durch Einführen von einem zusätzlichen Cystein, hergestellt. Das Peptid kann dabei entweder N-,C-terminal oder auch beliebig in der Sequenz mit einem sogenannten Linker modifiziert sein. Die Synthese der entsprechenden Peptide erfolgt wie in Beispiel 1 beschrieben.

Für die Umsetzung zum Polyhapten wird der NH₂-Gruppen-haltige Träger zuerst mit dem entsprechenden Aktivester des Labels und anschließend mit Maleinimidoalkyl-Gruppen, durch Behandlung vorzugsweise mit Maleinimidohexyl- (MHS) oder Maleinimidopropyl-N-hydroxysuccinimidoester (MPS), beladen. Dadurch werden die Aminogruppen der ε-Aminoseitenkette von Lysinresten im Protein z.T. mit Biotin, Digoxigenin oder Bipyridylruthenium gelabelt und zum anderen Teil in Maleinimidgruppen umgewandelt.

Die Umsetzung des Trägers mit den Aktivestern erfolgt vorzugsweise in 0,1 M Kaliumphosphatpuffer pH 7,0 - 8,5 und einer Konzentration von 5-20 mg/ml innerhalb von 2-4 h bei Raumtemperatur. Die niedermolekularen Bestandteile werden entweder über Dialyse oder Gelchromatographie (AcA 202-Gel, Eluent 0,1 M Kaliumphosphatpuffer pH 7-8,5) abgetrennt.

In einem weiteren Schritt wird dann das Peptid oder die Peptidmischung mit der reaktiven Mercaptofunktion an das MHS-modifizierte, gelabelte Trägerprotein in 0,1 M Kaliumphosphatpuffer pH 8,5 innerhalb von 6 h bei Raumtemperatur gekuppelt . Nicht umgesetztes Peptid wird entweder mittels Dialyse oder Gelchromatographie abgetrennt.

Die Polyhaptene können in Tests wie in Beispiel 2 beschrieben eingesetzt werden.

Soll der Label direkt am Peptid sitzen, wird das Polyhapten anlog synthetisiert, nur wird jetzt ein entsprechend gelabeltes, SH-aktiviertes Peptid eingesetzt.

## Patentansprüche

1. Verfahren zur Herstellung eines Peptidgemisches, worin man Peptide an einer Festphase aus Aminosäurederivaten synthetisiert und an mindestens einer Aminosäureposition für die Kopplungsreaktion ein Gemisch von Derivaten unterschiedlicher Aminosäuren verwendet,
**dadurch gekennzeichnet,**
daß nach jedem Kopplungsschritt, in dem ein Gemisch von Derivaten unterschiedlicher Aminosäuren eingesetzt wird, ein Reaktionsschritt durchgeführt wird, um an Peptidsequenzen, an die im vorherigen Kopplungsschritt keine Aminosäure gebunden worden ist, einen selektiven Kettenabbruch zu bewirken, und das Peptidgemisch nach der Synthese von der Festphase abgespalten wird.

2. Verfahren nach Anspruch 1,
**dadurch gekennzeichnet,**
daß man Peptidsequenzen synthetisiert, die einen reaktiven Epitopbereich sowie einen Spacerbereich umfassen.

3. Verfahren nach einem der vorhergehenden Ansprüche,
**dadurch gekennzeichnet,**
daß ein Gemisch aus Peptiden auf Basis einer der HIV I-, HIV Subtyp 0- oder HIV II-Aminosäuresequenzen 
NNTRKSISIG PGRAFYT (I)
NTTRSISIGP GRAFYT (II)
QIDIQEMRIG PMAWYS (III)
QARILAVERY LKDQQLLGIW GASG (IV)
LGIWGCSGKL ICTTAVPWNA SWS (V)
KDQQLLGIWG SSGKL (VI)
ALETLLQNQQ LLSLW (VII)
LSLWGCKGKL VCYTS (VIII)
WGIRQLRARL LALETLLQN (IX) und
QAQLNSWGCA FRQVCHTTVP WPNDSLT (X)
 oder einer Teilsequenz davon mit einer Länge von mindestens 6 Aminosäuren synthetisiert wird.

4. Peptidgemisch, erhältlich durch ein Verfahren nach einem der vorhergehenden Ansprüche, das mehrere Peptide enthält, die jeweils an selektiv ausgewählten Positionen mindestens eine Gruppe ausgewählt aus Markierungs-, Aktivierungs- und Festphasenbindegruppen aufweisen.

5. Peptidgemisch, das mehrere Peptide enthält, wobei die Peptide Varianten eines immunologisch reaktiven Eptitopbereichs darstellen und jeweils an selektiv ausgewählten Positionen eine Gruppe ausgewählt aus Markierungs- Aktivierungs- und Festphasenbindegruppen aufweisen.

6. Verfahren zur Herstellung einer Polyhapten-Zusammensetzung,
**dadurch gekennzeichnet,**
daß man ein Peptidgemisch nach einem der Ansprüche 4 bis 5 an einen Träger koppelt.

7. Polyhapten-Zusammensetzung, erhältlich durch ein Verfahren nach Anspruch 6.

8. Polyhapten-Zusammensetzung, umfassend einen Träger und daran kovalent gekoppelt ein Gemisch von Peptiden, wobei die Peptide Varianten eines immunologisch reaktiven Epitopbereichs darstellen und über eine selektiv ausgewählte Position mit dem Träger gekoppelt sind.

9. Verwendung eines Peptidgemisches nach einem der Ansprüche 4 oder 5 oder hergestellt nach einem der Ansprüche 1 bis 3 oder/und einer Polyhapten-Zusammensetzung nach einem der Ansprüche 7 oder 8 oder hergestellt nach Anspruch 6 als immunologisches Nachweisreagenz.

10. Verfahren zur immunologischen Bestimmung eines Analyten in einer Probeflüssigkeit,
**dadurch gekennzeichnet,**
daß man die Probeflüssigkeit mit einem Peptidgemisch nach einem der Ansprüche 4 oder 5 oder hergestellt nach einem der Ansprüche 1-3 oder/und mit einer Polyhapten-Zusammensetzung nach einem der Ansprüche 7 oder 8 oder hergestellt nach Anspruch 6 kontaktiert, wobei das Peptidgemisch oder/und das Polyhapten Peptide umfaßt, die spezifisch an den zu bestimmenden Analyten binden, und den Analyten über die Bildung von Immunkomplexen qualitativ oder/und quantitativ nachweist.

11. Reagenz zur immunologischen Bestimmung eines Analyten,
**dadurch gekennzeichnet,**
daß es mindestens ein gegen den zu bestimmenden Analyten gerichtetes Peptidgemisch nach einem der Ansprüche 4 oder 5 oder hergestellt nach einem der Ansprüche 1 - 3 oder/und ein Polyhapten nach einem der Ansprüche 7 oder 8 oder hergestellt nach Anspruch 6 umfaßt.


SEQUENZPROTOKOLL

(1) ALLGEMEINE ANGABEN:

(i) ANMELDER:
(A) NAME: Boehringer Mannheim GmbH
(B) STRASSE: Sandhofer Strasse 116
(C) ORT: Mannheim
(E) LAND: Deutschland
(F) POSTLEITZAHL: D-68305

(ii) BEZEICHNUNG DER ERFINDUNG: Verfahren zur Herstellung eines
Peptidgemisches

(iii) ANZAHL DER SEQUENZEN: 10

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 17 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 1:

Asn Asn Thr Arg Lys Ser Ile Ser Ile Gly Pro Gly Arg Ala Phe Tyr
1               5                   10                  15

Thr

(2) ANGABEN ZU SEQ ID NO: 2:

(i) SEQUENZKENNZEICHEN:
(A) LÄNGE: 16 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 2:

Asn Thr Thr Arg Ser Ile Ser Ile Gly Pro Gly Arg Ala Phe Tyr Thr
1               5                   10                  15

(2) ANGABEN ZU SEQ ID NO: 3:

(i) SEQUENZKENNZEICHEN:
(A) LÄNGE: 16 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 3:

Gln Ile Asp Ile Gln Glu Met Arg Ile Gly Pro Met Ala Trp Tyr Ser
1               5                   10                  15

(2) ANGABEN ZU SEQ ID NO: 4:

(i) SEQUENZKENNZEICHEN:
(A) LÄNGE: 24 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 4:

Gln Ala Arg Ile Leu Ala Val Glu Arg Tyr Leu Lys Asp Gln Gln Leu
1               5                   10                  15

Leu Gly Ile Trp Gly Ala Ser Gly
            20

(2) ANGABEN ZU SEQ ID NO: 5:

(i) SEQUENZKENNZEICHEN:
(A) LÄNGE: 23 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 5:

Leu Gly Ile Trp Gly Cys Ser Gly Lys Leu Ile Cys Thr Thr Ala Val
1               5                   10                  15

Pro Trp Asn Ala Ser Trp Ser
            20

(2) ANGABEN ZU SEQ ID NO: 6:

(i) SEQUENZKENNZEICHEN:
(A) LÄNGE: 15 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 6:

Lys Asp Gln Gln Leu Leu Gly Ile Trp Gly Ser Ser Gly Lys Leu
1               5                   10                  15

(2) ANGABEN ZU SEQ ID NO: 7:

(i) SEQUENZKENNZEICHEN:
(A) LÄNGE: 15 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 7:

Ala Leu Glu Thr Leu Leu Gln Asn Gln Gln Leu Leu Ser Leu Trp
1               5                   10                  15

(2) ANGABEN ZU SEQ ID NO: 8:

(i) SEQUENZKENNZEICHEN:
(A) LÄNGE: 15 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 8:

Leu Ser Leu Trp Gly Cys Lys Gly Lys Leu Val Cys Tyr Thr Ser
1               5                   10                  15

(2) ANGABEN ZU SEQ ID NO: 9:

(i) SEQUENZKENNZEICHEN:
(A) LÄNGE: 19 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 9:

Trp Gly Ile Arg Gln Leu Arg Ala Arg Leu Leu Ala Leu Glu Thr Leu
1               5                   10                  15

Leu Gln Asn

(2) ANGABEN ZU SEQ ID NO: 10:

(i) SEQUENZKENNZEICHEN:
(A) LÄNGE: 27 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 10:

Gln Ala Gln Leu Asn Ser Trp Gly Cys Ala Phe Arg Gln Val Cys His
1               5                   10                  15

Thr Thr Val Pro Trp Pro Asn Asp Ser Leu Thr
            20                  25